# EUROPEAN PATENT SPECIFICATION

(11) **EP 0 978 324 B1**
(45) Date of publication and mention of the grant of the patent: **30.03.2005**
(21) Application number: 99306063.1
(22) Date of filing: 30.07.1999
(51) Int. Cl.: B05D 3/14, B05D 7/24, A61L 33/06, A61L 27/34

(54) **Process for coating surfaces to enhance their biocompatibility**
Oberflächenbeschichtungsverfahren zur Verbesserung der Biokompatibilität
Procédé de revêtement de surface pour améliorer leur biocompatibilité

(30) Priority: 04.08.1998 EP 98480053; 10.02.1999 GB 9902824
(43) Date of publication of application: 09.02.2000
(73) Proprietor: Dow Corning France S.A., 06904 Sophia Antipolis (FR)
(72) Inventor: Colas, Andre Rudolf Louis, 096560 Valbonne (FR); Briquet, Francois Jean, Le Tamango, 006200 Nice (FR)
(74) Representative: Williams, Paul Edwin

(56) References cited:
- EP-A- 0 516 308
- WO-A-92/07464
- WO-A-98/08551
- US-A- 3 957 713
- US-A- 5 007 928

## Description

The present invention relates to methods of coating surfaces so that they are substantially inert and in particular are biocompatible.

Inert surfaces are required in a number of applications. In industrial plants, chemicals are passed through conduits and stored in storage facilities in contact with the walls of the vessels. Over a period of time, reactive chemicals may erode or leach into the surface of the walls, causing contamination of the chemical by material from the surface and loss of chemical into the walls. This may be a particular problem in, for example, the pharmaceutical industry. Similarly, containers or bottles used to hold or store these pharmaceutical chemicals should have resistant or inert surfaces.

Biocompatible surfaces are particularly important in medical apparatus. The term "apparatus" is used herein to encompass medical devices such as implants and prosthetics such as arterial valves which are in contact with the blood for extended periods of time as well as components such as tubing conduits, or other parts of an extracorporeal circulation circuit used, for example, in dialysis and cardio-surgery. The components used in these circuits are generally made of substantially inert polymeric materials such as plastic or elastomeric materials with varying levels of biompatibility. Consequently, they may be involved in adverse biological reactions. Such reactions may lead to a postperfusion syndrome experienced by certain patients.

As the biocompatibility of these apparatus is mainly a result of their specific surface properties, changing the surface composition, for example, by applying or grafting on more biocompatible material may enhance their biocompatibility and improve the final patient comfort.

The use of plasma deposition techniques to apply coatings and to otherwise modify surfaces is well known. Although the precise details of the processes vary, in general terms, coatings are produced by placing the substrate to be coated within a reactor vessel at low pressure and subjecting it to a plasma discharge. The effect that this has on the surface depends upon the gaseous material present within the reactor during the plasma discharge.

When the reactor contains an inert gas, plasma discharge can lead to an activation of the surface, where free radicals are generated on the surface. In this state, subsequent exposure to a reagent can produce a reaction where the reagent becomes grafted to the surface. Polymerisation reactions may also take place depending upon the nature of the reagent and the condition of the surface.

If the surface is subjected to a plasma discharge for an extended period of time in the presence of an inert gas, it will become etched. Reagents may subsequently be grafted onto the surface, optionally under the influence of a plasma discharge.

Where active reagents such as one or more monomeric compounds are present in the reaction vessel during the plasma discharge, free radical-containing moieties or other intermediates may be generated, leading to polymerisation or co-polymerisation as well as grafting to the surface. The substrate in this case therefore has a polymeric material grafted onto the surface. This process is known as "plasma polymerisation".

Attempts have been made to improve the biocompatibility of the surfaces in particular of medical devices, using plasma discharge techniques. For example, US Patent No. 4656083 describes a plasma discharge process in which a fluorocarbon layer is deposited onto the surface of a biomedical device, in order to enhance its biocompatibility.

A method for pretreating the surface of a medical device so as to deposit a polymeric adhesive layer using plasma polymerisation or grafting methods is described in US Patent No. 5451428. In this process, a biological coating and in particular an anti-thrombogenic material is subsequently applied in a further step and adhers to the deposited adhesive layer.

US Patent No 5326584 describes a method of permanently modifying the surface of a substrate material by using plasma etching followed by a grafting of a material which is similar to the substrate material. The grafting process is induced by a second radio frequency plasma. It is suggested that this method could be employed to modify the surface of silicone elastomers by grafting hexamethyldisiloxane onto a previously etched surface.

The applicants have found that by depositing certain organosilicon materials onto surfaces using plasma discharge techniques, inert or biocompatible surfaces can be formed.

In one aspect, the invention provides a method of applying an inert coating to a surface, said method comprising in a first step, subjecting the surface to a plasma discharge in the presence of an inert gas for a period sufficient to activate the surface, and in a subsequent step, contacting the activated surface with a gaseous organosilicon material which reacts with the activated surface in the absence of plasma so as to deposit said organosilicon material on the surface.

The organosilicon material on the surface may be continuous or discontinuous but is sufficient to reduce the reactivity of the surface and increase its biocompatibility.

Suitable gases for use in the first stage of this process are inert gases, such as argon, neon, krypton or xenon, as well as other gases such as nitrogen or carbon dioxide, and also oxygen. Preferably an inert gas, in particular argon is used. In order to activate the surface, it is suitably exposed to the plasma gas discharge for a period of between about 5 seconds to about 30 minutes.

The plasma discharge process of the first step of the method of the invention is suitably effected in a reactor equipped with a radio frequency generator capable of generating a frequency of about 1 MHz to about 40 MHz and preferably 13.56 MHz. Alternatively, a microwave generator generating a frequency of about 233 MHz to about 466 MHz can be used. The power delivered is about 25 W to about 200 W and preferably about 100 W.

Suitable organosilicon materials used in the second step of the process of the invention are materials which, when in a gaseous form, react with the activated surface produced in the first step of the method of the invention to deposit the organosilicon material on the surface. Typically, this reaction occurs in the absence of plasma by way of a functional group such as hydrogen or an unsaturated group attached to a silicon.

The organosilicon materials can be gaseous at ambient temperature or they can be converted to a vapor by heating to temperatures up to 200°C, and preferably 50°C or less. Preferably, the organosilicon materials have a vapor pressure of at least 0.1Hgmm at room temperature.

Examples of suitable organosilicon materials include organosilanes and organosiloxanes. Examples of organosilanes include materials of formula (I):

R¹ ₙ SiR² ₙ (I)

wherein each group R¹ is independently selected from the group consisting of hydrogen or optionally substituted alkenyl; each group R² is independently selected from an optionally substituted alkyl group of 1 to 20 carbon atoms; or a group (OR³) or (OSiR³₃) , where each R³ is independently an optionally substituted alkyl group of 1 to 20 carbon atoms; n is an integer of 1 to 3; m is an integer of 1 to 3 and n + m is 4. Specific silanes can contain the same or different R¹ groups, e.g., vinyl and hexenyl groups, and/or different R² groups, e.g., methyl groups and (OSiR³₃)groups.

Examples of organosiloxanes include materials of the structure (II)

R¹ ₐR² ₍₃₋ₐ₎Si(OSiR⁴ ₂)ₓOSiR¹ _{b}R² _{(3-b)} (II)

wherein each R¹ and R² are as defined above, each group R⁴ is independently selected from the group consisting of hydrogen, optionally substituted alkenyl groups; optionally substituted alkyl groups of 1 to 20 carbon atoms and aryl groups, with the proviso that at least one R¹ or R⁴ group per molecule is an unsubstituted alkenyl group or a hydrogen; a is 0, 1, 2, or 3 and b is 0, 1, 2, or 3,x is 0 or a positive integer, for example of from 1 to 20. Again, the organosiloxanes can contain the same or different R¹ groups, e.g., vinyl and hexenyl groups, the same or different R² groups, e.g., methyl groups and (OR³) groups, and/or the same or different R⁴ groups, e.g., methyl groups and phenyl groups. In a preferred embodiment, one R¹ group per molecule is an optionally substituted alkenyl, R² is an optionally substituted alkyl, and R⁴ is an optionally substituted alkyl such that the structure comprises

R¹SiR² ₂(OSiR⁴ ₂)ₓOSiR² ₃

In a more preferred embodiment, the R² and R⁴ groups of this structure comprise substantially all methyl groups.

As used herein, the term "alkyl" refers to straight or branched chain groups, for example of from 1 to 20, suitably from 1 to 3 carbon atoms in length. The term "alkenyl" refers to straight or branched unsaturated hydrocarbon groups which include at least one carbon-carbon double bond and suitably from 2-20, preferably from 2-6 carbon atoms. Terms such as "alkoxy" will be interpreted as meaning an O-alkyl group as is conventional.

Particularly suitable unsubstituted alkenyl groups R¹ include vinyl, hexenyl or allyl. A preferred group is vinyl.

Suitable optional substitutents for alkyl and alkenyl groups R¹, R^{2,} R³ and R⁴ include halogen such as fluoro, preferably in the form of a perhaloalkyl group such as trifluoromethyl, oxo, alkoxy, amino or mono- or di-alkyl amino, or a silicon derivative such as an alkyl silane. A particular example of such a substituted alkyl group is 3,3,3-trifluoropropyl.

Particularly preferred groups for R², R³ and/or R⁴ are C₁₋₃ alkyl groups, such as methyl.

In a particularly preferred embodiment, at least one R¹ group or, where appropriate, R⁴ is hydrogen or vinyl.

Thus specific examples of compounds of organosilicon materials for use in the invention include vinylmethylbisEtrimethylsiloxy)silane or vinylpentamethyldisiloxane, vinylheptamethyltrisiloxane, vinyl tris (trimethylsiloxy) silane, trimethylsilane, and vinyltrimethylsilane, preferably vinylmethylbis (trimethylsiloxy)silane or vinylpentamethyldisiloxane.

If necessary or desired, the organosilicon materials can be diluted in a carrier gas for delivery to the activated surface. Suitable carriers include, for example, inert gases such as argon.

The surfaces of materials suitable for use in the method of the invention are those which are able to support free radicals such as plastics and polymers. These materials include polycarbonates (PC), polyurethanes (PU), polypropylenes (PP), polyethylenes (PE)(including polytetrafluoroethylenes),polyvinylchloride (PVC), silicone elastomers, polyesters (such as polyethylene tetraphthalate), polymethylmethacrylate (PMMA) and polymers of hydroxyethylmethacrylate. Preferred polymeric materials include silicone elastomers, PC, PVC, PU, PP, PE and PMMA.

Coatings derived from the deposition process of the present invention may comprise the organosilicon material directly attached to the surface or the organosilicon material or it may partially or fully polymerize prior to or during deposition. These coatings are often inert to many chemical reagents and therefore have a range of utilities where reaction between these reagents and surfaces is to be avoided. In particular however, the coatings described above have good biocompatibility. Thus they are particularly suitable for application to medical apparatus as outlined above, in particular to tubing and other components used in extracorporeal circulation circuits. Such apparatus will, in general, comprise the elastomeric or rigid plastics material outlined above.

Organosilicon coatings applied using this process improve significantly both the biocompatibility and the thromboresistance of the treated materials.

Coated surfaces obtainable by the method as described above, and articles which comprise such surfaces form a further aspect of the invention.

In a preferred embodiment, the article in the method of the present invention is a medical apparatus

The apparatus suitably comprises an elastomeric or rigid plastics material which provides the substrate for the said inert coating. Examples of such apparatus include medical devices like an implant or prosthesis for introduction into the body of a patient, or a component such as tubing used in extracorporeal circulation circuits

The invention will now be particularly described by way of Example.

### Example 1

### Treatment of Medical Plastic

A series of three tests were carried out, each using a different slab of medical grade PC plastic (44x10x1mm in size). Each slab was placed individually in a radio frequency plasma reactor (IPC Branson -20L) which uses a frequency of about 13.56 MHz at a power of about 100W. In the first step, the slabs were subjected to a plasma treatment in the presence of argon gas at 25°C and atmospheric pressure for a period of 15 seconds in order to activate the surface.

In two cases, a gaseous organosilicon material was then introduced into the reaction chamber. In a control experiment, only nitrogen was introduced. Thus the second step treatment can be summarised as follows:

| Slab No | Compound Applied |
|---|---|
| 1 | Vinylmethylbis (trimethylsiloxy)silane |
| 2 | Vinylpenta-methyldisiloxane |
| 3 | Nitrogen gas only (control) |

In the case of the volatile organosilicon material, the gaseous reagent was produced by heating the corresponding liquid at 50°C, and the gaseous product pushed towards the surface of the slab in the reactor using nitrogen gas. Each slab was exposed to the relevant vapours/and or gases for a period of 45 minutes at ambient temperature.

### Example 2

### Biocompatibility of Treated surfaces

The biocompatibility improvement of the slabs obtained in Example 1 was investigated using a coagulation test with bovine bloods in contact after total immersion of the untreated and treated materials. This test assesses globally the activation of the coagulation cascade which is a key parameter when it comes to evaluate the hemocompatibility of a material. The higher the coagulation time, the lower the coagulation activation, thus the better the biocompatibility of the material.

The results, are expressed as a percentage of the coagulation time of the PC control, are shown in the following Table.

| Slab No. | Donor 1 | Donor 2 |
|---|---|---|
| 1 | 128 | 128 |
| 2 | 150 | - |
| 3 (control) | 100 | 100 |

Using blood from 2 different donors, the coagulation time of the untreated material was increased by 28% and up to 50% depending on the material used. This significant increase of the coagulation values with the coated material corresponds to a significantly lower thrombogenicity and higher biocompatibility.

## Claims

1. A method of applying an inert coating to a surface, said method comprising in a first step, subjecting the surface to a plasma discharge in the presence of an inert gas for a period sufficient to activate the surface, and in a second step, contacting the activated surface with a gaseous organosilicon material in the absence of plasma so as to deposit said organosilicon material on the surface.

2. A method according to claim 1 wherein the inert gas is argon.

3. A method according to claims 1 or 2 wherein in the first step, the surface is exposed to plasma discharge for a period of between about 5 seconds to about 30 minutes.

4. A method according to any one of claims 1 to 3 wherein said gaseous organosilicon material is gaseous at ambient temperature, or can be converted to a vapour by heating to temperatures of up to 200°C.

5. A method according to any one of the preceding claims wherein the gaseous organosilicon material is a compound of formula (I)
R¹ ₙ SiR² ₘ (I)
wherein each group R¹ is independently selected from the group consisting of hydrogen or optionally substituted alkenyl; each group R² is independently selected from an optionally substituted alkyl group of 1 to 20 carbon atoms or a group (OR³) or (OSIR³₃) where each R³ is independently an optionally substituted alkyl group of 1 to 20 carbon atoms; n is an integer of 1 to 3; m is an integer of 1 to 3 provided that n + m is 4;
or a compound of formula (II)
R¹ ₐR² ₍₃₋ₐ₎Si(OSiR⁴ ₂)ₓOSiR¹ _{b}R² _{(3-b)} (II)
wherein each R¹ and R² are as defined above, each group R⁴ is independently selected from the group consisting of hydrogen, optionally substituted alkenyl, and optionally substituted alkyl groups of 1 to 20 carbon atoms or an aryl group, with the proviso that at least one R¹ or R⁴ group per molecule is an unsubstituted alkenyl group or a hydrogen; a is 0, 1, 2, or 3, b is 0, 1, 2 or 3 and x is 0 or a positive integer.

6. A method according to claim 5 wherein at least one R¹ in formula (I) or at least one group R¹ or R⁴ group in formula (II) is hydrogen, vinyl, hexenyl or allyl.

7. A method according to claim 6 wherein said group R¹ or R⁴ group is vinyl.

8. A method according to any one of claims 5 or claim 7 wherein at least some groups R², R³ and/or R⁴ in formula (I) or (II) are C₁₋₃ alkyl groups.

9. A method according to anyone of the preceding claims wherein the surface is a plastic or an elastomeric material.

10. A method according to claim 9 wherein the plastic or elastomeric material comprises a polycarbonate (PC), a polyurethane (PU), a polypropylene (PP), a polyethylene (PE), polyvinylchloride (PVC), a silicone elastomer, a polyester, a polymethylmethacrylate (PMMA), or a polymer of hydroxyethylmethacrylate.

11. A method according to claim 10 wherein the polymeric material is a silicone elastomer, PC, PU, PP, PVC, PE or PMMA.

12. A method according to any of the preceding claims, wherein the surface forms part of an article.

13. A method according to claim 12 wherein the article is a medical apparatus.

14. A method according to claim 13 wherein the medical apparatus has its surface as a rigid plastics or elastomeric material.

15. A method according to claim 13 or 14, wherein the apparatus comprises an implant or prosthesis for introduction into the body of a patient, or a component used in extracorporeal circulation circuits.

## Patentansprüche

1. Verfahren zur Aufbringung einer inerten Beschichtung auf eine Oberfläche, wobei dieses Verfahren in einem ersten Schritt Aussetzen der Oberfläche an eine Plasmaentladung in Gegenwart eines Inertgases für einen ausreichenden Zeitraum, um die Oberfläche zu aktivieren, und in einem zweiten Schritt In-Berührung-Bringen der aktivierten Oberfläche mit einem gasförmigen Organosiliciummaterial in Abwesenheit von Plasma, um dieses Organosiliciummaterial auf der Oberfläche abzuscheiden, umfasst.

2. Verfahren nach Anspruch 1, wobei das Inertgas Argon ist.

3. Verfahren nach Anspruch 1 oder 2, wobei in dem ersten Schritt die Oberfläche Plasmaentladung für einen Zeitraum zwischen etwa 5 s bis etwa 30 min ausgesetzt wird.

4. Verfahren nach einem der Ansprüche 1 bis 3, wobei dieses gasförmige Organosiliciummaterial bei Umgebungstemperatur gasförmig ist oder durch Erwärmen auf Temperaturen bis zu 200°C in einen Dampf umgewandelt werden kann.

5. Verfahren nach einem der vorstehenden Ansprüche, wobei das gasförmige Organosiliciummaterial eine Verbindung der Formel (I)
R¹ ₙSiR² ₘ (I),
worin jede Gruppe R¹ unabhängig voneinander ausgewählt ist aus der Gruppe bestehend aus Wasserstoff oder optional substituiertem Alkenyl; jede Gruppe R² unabhängig voneinander ausgewählt ist aus einer optional substituierten Alkylgruppe mit 1 bis 20 Kohlenstoffatomen oder einer Gruppe (OR³) oder (OSiR³₃), worin jedes R³ unabhängig voneinander eine optional substituierte Alkylgruppe mit 1 bis 20 Kohlenstoffatomen ist; n eine ganze Zahl von 1 bis 3 ist; m eine ganze Zahl von 1 bis 3 ist, vorausgesetzt, dass n+m gleich 4 ist;
oder eine Verbindung der Formel (II)
R¹ₐR²₍₃₋ₐ₎Si(OSiR⁴₂)ₓOSiR¹_{b}R²_{(3-b)} (II)
ist, worin jedes R¹ und R² wie oben definiert ist, jede Gruppe R⁴ unabhängig voneinander ausgewählt ist aus der Gruppe bestehend aus Wasserstoff, optional substituierten Alkenyl- und optional substituierten Alkylgruppen mit 1 bis 20 Kohlenstoffatomen oder einer Arylgruppe, unter der Voraussetzung, dass mindestens eine R¹- oder R⁴-Gruppe pro Molekül eine unsubstituierte Alkenylgruppe oder ein Wasserstoff ist; a gleich 0, 1, 2 oder 3 ist, b gleich 0, 1, 2 oder 3 ist und x gleich null oder eine positive ganze Zahl ist.

6. Verfahren nach Anspruch 5, wobei mindestens ein R¹ in Formel (I) oder mindestens eine Gruppe R¹ oder R⁴ in Formel (II) gleich Wasserstoff, Vinyl, Hexenyl oder Allyl ist.

7. Verfahren nach Anspruch 6, wobei diese Gruppe R¹ oder R⁴ gleich Vinyl ist.

8. Verfahren nach einem der Ansprüche 5 oder 7, wobei mindestens einige Gruppen R², R³ und/oder R⁴ in Formel (I) oder (II) C₁₋₃-Alkylgruppen sind.

9. Verfahren nach einem der vorstehenden Ansprüche, wobei die Oberfläche ein plastisches oder elastomeres Material ist.

10. Verfahren nach Anspruch 9. wobei das plastische oder elastomere Material ein Polycarbonat (PC), ein Polyurethan (PU), ein Polypropylen (PP), ein Polyethylen (PE), Polyvinylchlorid (PVC), ein Siliconelastomer, ein Polyester, ein Polymethylmethacrylat (PMMA) oder ein Polymer von Hydroxyethylmethacrylat umfasst.

11. Verfahren nach Anspruch 10, wobei das polymere Material ein Siliconelastomer, PC, PU, PP, PVC, PE oder PMMA ist.

12. Verfahren nach einem der vorstehenden Ansprüche, wobei die Oberfläche Teil eines Gegenstandes bildet.

13. Verfahren nach Anspruch 12, wobei der Gegenstand ein medizinisches Gerät ist.

14. Verfahren nach Anspruch 13, wobei das medizinische Gerät seine Oberfläche als ein starres plastisches oder elastomeres Material aufweist.

15. Verfahren nach Anspruch 13 oder 14, wobei das Gerät ein Implantat oder eine Prothese zur Einführung in den Körper eines Patienten oder eine Komponente, die in extrakorporalen Zirkulationskreisläufen verwendet wird, umfasst.

## Revendications

1. Méthode d'application d'un revêtement inerte sur une surface, ladite méthode comprenant, dans une première étape, la soumission de la surface à une décharge de plasma en présence d'un gaz inerte pendant une durée suffisante pour activer la surface et, dans une seconde étape, la mise en contact de la surface activée avec une matière gazeuse organosiliciée en l'absence de plasma de manière à déposer ladite matière organosiliciée sur la surface.

2. Méthode selon la revendication 1, dans laquelle le gaz inerte est l'argon.

3. Méthode selon les revendications 1 ou 2, dans laquelle, dans la première étape, la surface est exposée à une décharge de plasma pendant une durée entre environ 5 secondes et environ 30 minutes.

4. Méthode selon l'une quelconque des revendications 1 à 3, dans laquelle ladite matière organosiliciée gazeuse est gazeuse à la température ambiante ou peut être convertie en une vapeur par chauffage à des températures allant jusqu'à 200°C.

5. Méthode selon l'une quelconque des revendications précédentes, dans laquelle la matière organosiliciée gazeuse est un composé de formule (I)
R¹ ₙSiR² ₘ (I)
où chaque groupe R¹ est choisi de façon indépendante dans le groupe constitué par l'hydrogène ou un alcényle substitué de façon optionnelle ; chaque groupe R² est choisi de façon indépendante parmi un groupe alkyle de 1 à 20 atomes de carbone substitué de façon optionnelle ou un groupe (OR³) ou (OSiR³₃), où chaque R³ est, de façon indépendante, un groupe alkyle ayant 1 à 20 atomes de carbone substitué de façon optionnelle ; n est un entier de 1 à 3 ; m est un entier de 1 à 3 sous réserve que n + m soit égal à 4 ;
ou un composé de formule (II)
R¹ ₐR² ₍₃₋ₐ₎Si(OSiR⁴ ₂)ₓOSiR¹ _{b}R² _{(3-b)} (II)
où chaque R¹ et R² est tel que défini ci-dessus, chaque groupe R⁴ est choisi de façon indépendante dans le groupe constitué par l'hydrogène, des groupes alcényle substitués de façon optionnelle, et des groupes alkyle ayant 1 à 20 atomes de carbone substitués de façon optionnelle ou un groupe aryle, sous réserve qu'au moins un groupe R¹ ou R⁴ par molécule soit un groupe alcényle non substitué ou un hydrogène ; a est égal à 0, 1, 2 ou 3 et b est égal à 0, 1, 2 ou 3, x est égal à 0 ou est un entier positif.

6. Méthode selon la revendication 5, dans laquelle au moins un R¹ dans la formule (I) ou au moins un groupe R¹ ou un groupe R⁴ dans la formule (II) est un hydrogène, un vinyle, un hexényle ou un allyle.

7. Méthode selon la revendication 6, dans laquelle ledit groupe R¹ ou ledit groupe R⁴ est un vinyle.

8. Méthode selon l'une quelconque des revendications 5 ou la revendication 7, dans laquelle au moins certains groupes R², R³ et/ou R⁴ dans la formule (I) ou (II) sont des groupes alkyle en C₁₋₃.

9. Méthode selon l'une quelconque des revendications précédentes dans laquelle la surface est une matière plastique ou une matière élastomère.

10. Méthode selon la revendication 9, dans laquelle la matière plastique ou la matière élastomère comprend un polycarbonate (PC), un polyuréthane (PU), un polypropylène (PP), un polyéthylène (PE), un chlorure de polyvinyle (PVC), un élastomère de silicone, un polyester, un polyméthacrylate de méthyle (PMMA) ou un polymère du méthacrylate d'hydroxyéthyle.

11. Méthode selon la revendication 10, dans laquelle la matière polymère est un élastomère de silicone, PC, PU, PP, PVC, PE ou PMMA.

12. Méthode selon l'une quelconque des revendications précédentes, dans laquelle la surface constitue une partie d'un article.

13. Méthode selon la revendication 12, dans laquelle l'article est un appareil médical.

14. Méthode selon la revendication 13 dans laquelle la surface de l'appareil médical est sous la forme d'une matière plastique rigide ou d'une matière élastomère.

15. Méthode selon la revendication 13 ou 14, dans laquelle l'appareil comprend un implant ou une prothèse destinés à être introduits dans le corps d'un patient, ou un composant utilisé dans des circuits de circulation extracorporelle.
